## Europäisches Patentamt

## European Patent Office

## Office européen des brevets

(19)

(11) Veröffentlichungsnummer: **0 148 481**
**B1**

(12) **EUROPÄISCHE PATENTSCHRIFT**

(45) Veröffentlichungstag der Patentschrift:
**09.03.88**

(21) Anmeldenummer: **84115994.0**

(22) Anmeldetag: **20.12.84**

(51) Int. Cl.⁴: **C 07 C 21/12**, C 07 C 17/42

(54) Verfahren zum Stabilisieren von cyclohexenoxidhaltigem Perchlorethylen.

(30) Priorität: **22.12.83 DE 3346530**

(43) Veröffentlichungstag der Anmeldung:
**17.07.85 Patentblatt 85/29**

(45) Bekanntmachung des Hinweises auf die Patenterteilung:
**09.03.88 Patentblatt 88/10**

(84) Benannte Vertragsstaaten:
**AT BE DE FR GB IT NL**

(56) Entgegenhaltungen:
**EP - A - 0 111 894**
**DE - A - 2 853 848**

**Die Akte enthält technische Angaben, die nach dem Eingang der Anmeldung eingereicht wurden und die nicht in dieser Patentschrift enthalten sind.**

(73) Patentinhaber: **WACKER-CHEMIE GMBH,
Prinzregentenstrasse 22, D-8000 München 22 (DE)**

(72) Erfinder: **Blum, Klaus, Dr. Dipl.-Chem., Amatiweg 5,
D-8263 Burghausen (DE)**
Erfinder: **Strasser, Rudolf, Dr. Dipl.-Chem.,
Lindacherstrasse 58, D-8263 Burghausen (DE)**

## Beschreibung

Hochstabilisiertes Perchlorethylen, das u.a. zur Metallentfettung eingesetzt wird, enthält neben Antioxidantien und Metalldeaktivatoren vielfach Cyclohexenoxid als Säurebindemittel. Beim Gebrauch von stabilisiertem Perchlorethylen werden nun − zumindest in Spuren − Lewissäuren, wie Zinkchlorid, Eisenchlorid, Aluminiumchlorid und andere eingeschleppt. Diese Lewissäuren katalysieren die Umwandlung von Cyclohexenoxid zu dem für Stabilisierungszwecke wirkungslosen Cyclopentylaldehyd. Der dadurch hervorgerufene beschleunigte Abbau von Cyclohexenoxid destabilisiert letztlich das in Gebrauch befindliche Perchlorethylen.

Es wurde deshalb bereits gemäss DE-OS-2 853 848 vorgeschlagen, Cyclohexenoxid mit 2.3-Epoxipropanol gegen die Umwandlung in Cyclopentylaldehyd zu stabilisieren. Nachteiligerweise neigt 2.3-Epoxipropanol zu Polykondensationsreaktionen, wobei Produkte entstehen, die sich als schmierige Beläge am Reinigungsgut und an den Wänden der Reinigungskammern absetzen. Beispielsweise werden durch derartige Ablagerungen die Widerstandskenndaten gereinigter Schaltelemente derart stark verändert, dass diese funktionsuntüchtig werden.

Aufgabe der Erfindung war es, Substanzen aufzufinden, die Cyclohexenoxid, das als Stabilisierungsmittel im Perchlorethylen enthalten ist, gegen die Umlagerung in Cyclopentylaldehyd zu stabilisieren. An diese Substanzen musste ferner die Anforderung gestellt werden, dass sie nicht ihrerseits die Ursache sind für die Bildung von Verfärbungen oder Belägen am Reinigungsgut, insbesondere auch an zu reinigenden Kupfer- und Messingteilen.

Es wurde nun gefunden, dass die genannten Anforderungen von 4-Vinyl-cyclohexenmonoxid, 7.8-Epoxiocten-1 und 1.2-Epoxicycloocten-5 erfüllt werden.

Gegenstand der Erfindung ist somit ein Verfahren zum Stabilisieren von Perchlorethylen, das als Stabilisierungsmittel Cyclohexenoxid enthält, das dadurch gekennzeichnet ist, dass dem Perchlorethylen 4-Vinyl-cyclohexenmonoxid und/oder 7.8-Epoxiocten-1 und/oder 1.2-Epoxicycloocten-5 in Mengen von 0,5 bis 10 Gew.-%, insbesondere in Mengen von 1 bis 4 Gew.-%, bezogen auf die Menge im Perchlorethylen anwesenden Cyclohexenoxids, zugesetzt werden.

4-Vinyl-cyclohexenmonoxid, 7.8-Epoxiocten-1 und 1.2-Epoxicycloocten-5 sind an sich bekannte Substanzen. Sie sind z.B. durch übliche Epoxidierungsreaktionen von 4-Vinyl-cyclohexen bzw. 1.7-Octadien bzw. 1.5-Cyclooctadien zugänglich.

Cyclohexenoxid wird in der Regel in Mengen von 1000 bis 5000 Gew. ppm, insbesondere in Mengen von 2000 bis 3000 Gew. ppm, jeweils bezogen auf das Gesamtgewicht des stabilisierten Perchlorethylens, eingesetzt.

Entsprechend ergibt sich, bezogen auf das Gesamtgewicht des erfindungsgemäss stabilisierten Perchlorethylens nach der bevorzugten Ausführungsform des Verfahrens eine Menge von 50 bis 500 Gew. ppm, insbesondere 80 bis 150 Gew. ppm an 4-Vinyl-cyclohexenmonoxid und/oder 7.8-Epoxiocten-1 und/oder 1.2-Epoxicycloocten-5. Der Zusatz der erfindungsgemäss einzusetzenden Epoxide erfolgt in der gleichen Weise, wie auch bereits bisher Epoxide dem zu stabilisierenden Perchlorethylen zugesetzt wurden.

Erfindungsgemäss stabilisiertes Perchlorethylen kann daneben auch jene stabilisierenden Zusätze enthalten, die auch bisher zur Stabilisierung von Perchlorethylen eingesetzt werden konnten. Es sind dies insbesondere

Amine, wie Triethylamin, Di-iso-propylamin, Di-methylisobutylamin, sec.-Butylamin, Pentylamin, Isopentylamin, 5-Methyl-2-hexan-amin, Di-iso-butylamin, N-methylpyrrol, N-methylmorpholin, u.a.

Ether, insbesondere Dialkylether, wie Dibutylether und Di-sec.-butylether, Dialkoximethane, wie Dimethoximethan und Diethoximethan, Glykoldialkylether, wie Butyl-glykol-tert.-butylether, Polyglykolether, wie Diglykol-tert.-butylether, Methoxi-diglykol- tert.-butylether, Triglykoldimethylether, Arylether, wie Diphenylether, Aralkylether, wie Dibenzylether, Arylalkylether, wie Anisol, Hydrochinondimethylether,

Olefine, wie Di-iso-butylen und Cycloheptatrien,

Alkylphenole, wie p-Kresol, o-Kresol, 2.6-Dimethylphenol, 2.4.6-Trimethylphenol, p-Iso-propylphenol, p-tert.-Butylphenol, 2.6-Di-tert.-Butylphenol, p-Amylphenol.

In dem erfindungsgemäss hergestellten Perchlorethylen liegt eine Stabilisatorkombination vor, bei der die destabilisierende Umlagerungsreaktion von Cyclohexenoxid in Cyclopentylaldehyd auch in Gegenwart von Lewissäuren weitgehend unterdrückt wird. Es gelingt überraschenderweise durch Zusatz von lediglich geringen Mengen an den beschriebenen Epoxiden, das Säurebindevermögen von in Gebrauch befindlichem Perchlorethylen über eine grössere Zeitspanne zu erhalten, Korrosionsschäden am Reinigungsgut zu vermeiden und die Bildung von Ablagerungen am Reinigungsgut zu verhindern.

Die Erfindung wird nun anhand von Beispielen und Vergleichsbeispielen näher erläutert:

Beispiel 1

Perchlorethylen mit einem Gehalt von
100 Gew. ppm 4-Vinylcyclohexenmonoxid
2500 Gew. ppm Cyclohexenoxid
15 Gew. ppm Diisopropylamin
25 Gew. ppm 2.4-Di-tert.-butylphenol
50 Gew. ppm N-methylpyrrol
wurde mit 200 Gew. ppm wasserfreiem Zinkchlorid versetzt und 72 Stunden am Rückfluss gekocht.

Das Säurebindevermögen wurde durch die Salzsäureadditionsfähigkeit nach der folgenden Methode gemessen:

Als Hydrochlorierungsreagens diente eine Lösung von Salzsäure in Isopropanol (4,4 cm³ kon-

zentrierte Salzsäure zu 500 cm³ ergänzt mit Isopropanol). Der Titer dieser Lösung wurde mit 0,1 N-Natronlauge gegen Bromphenolblau ermittelt (Verbrauch A).

Es wurden nun 25 cm³ Hydrochlorierungsreagens, 10 cm³ des oben beschriebenen Perchlorethylens und 25 cm³ Isopropanol geschüttelt und 10 Minuten bei Raumtemperatur stehengelassen. Danach wurde mit 0,1 N-Natronlauge gegen Bromphenolblau zurücktitriert (Verbrauch B).

Das Säurebindevermögen wurde nach der folgenden Formel berechnet:

$$\frac{(A-B) \times 4.0 \times 100 \times F}{V \times D \times 1000} = \text{Gew.-\% NaOH}$$

4.0 = Wirkungswert der 0,1 N-Natronlauge in mg/cm³

F = Faktor der 0,1 N-Natronlauge V = Volumen des eingesetzten Perchlorethylens in cm³ D = Dichte des Perchlorethylens in g/cm³

Ergebnis:
Das Säurebindevermögen betrug 0,086 Gew.-% NaOH

Vergleichsbeispiel 1
Es wurde die Arbeitsweise gemäss Beispiel 1 wiederholt, mit der Abänderung, dass ein ansonsten gleichermassen stabilisiertes Perchlorethylen ohne Zusatz von 4-Vinyl-cyclohexenmonoxid getestet wurde.

Ergebnis:
Das Säurebindevermögen betrug 0 Gew.-% NaOH.
Dieses Ergebnis demonstriert die destabilisierende Wirkung der Lewissäure Zinkchlorid, die durch die erfindungsgemässe Zugabe von 4-Vinyl-cyclohexenmonoxid (gemäss Beispiel 1) abgefangen wird.

Beispiel 2
Die Arbeitsweise gemäss Beispiel 1 wurde wiederholt, mit der Abänderung, dass anstatt 100 Gew. ppm 4-Vinyl-cyclohexenmonoxid, 100 Gew. ppm 7.8-Epoxiocten-1 zugesetzt wurden.
Das Säurebindevermögen betrugt 0,07 Gew.-% NaOH.

Beispiel 3
Die Arbeitsweise gemäss Beispiel 1 wurde wiederholt, mit der Abänderung, dass anstatt 100 Gew. ppm 4-Vinyl-cyclohexenmonoxid, 100 Gew. ppm 1.2-Epoxicycloocten-5 eingesetzt wurden.
Das Säurebindevermögen betrug 0,06 Gew.-% NaOH.

Beispiel 4
Es wurden jeweils Proben von gemäss den Beispielen 1, 2 und 3 stabilisiertem Perchlorethylens 72 Stunden am Rückfluss gekocht und anschliessend 10 mal destilliert. Danach wurde jeweils das Säurebindevermögen des 10. Destillats nach der gemäss Beispiel 1 beschriebenen Methode bestimmt und mit einer nichtdestillierten Probe verglichen.

Ergebnisse:

| | Säurebindevermögen in Gew.-% NaOH | |
| | vor Versuchs-beginn | 10. De-stillat |
| --- | --- | --- |
| Probe gemäss Beispiel 1 | 0.109 | 0.091 |
| Probe gemäss Beispiel 2 | 0.109 | 0.092 |
| Probe gemäss Beispiel 3 | 0.110 | 0.090 |

Das Säurebindevermögen bleibt auch nach der oben beschriebenen Destillationsprozedur bis zu etwa 90% erhalten. Es zeigt sich, dass die erfindungsgemässe Stabilisatorkombination nicht durch Destillation entmischt wird und so den in der Praxis zu stellenden Anforderungen gerecht wird.

Beispiel 5
Es wurden jeweils Proben des gemäss den Beispielen 1, 2 und 3 stabilisierten Perchlorethylens in ein verschliessbares Gefäss gebracht und innerhalb dieses Gefässes ein blankpolierter Kupfer-Metallstreifen so angeordnet, dass er zur Hälfte in die Flüssigkeit tauchte. Danach wurden die Gefässe verschlossen und 1 Stunde auf eine Temperatur von 80°C gehalten.
Auch nach dieser Prozedur blieben die Kupfer-Metallstreifen unverändert.

Vergleichsbeispiel 2
Die Arbeitsweise gemäss Beispiel 5 wurde wiederholt, mit der Abänderung, dass das gemäss Vergleichsbeispiel 1 stabilisierte Perchlorethylen verwendet wurde.
Der Kupferstreifen wies einen klebrigen Belag auf und war ankorrodiert.

Beispiel 6
Proben des gemäss den Beispielen 1, 2 und 3 stabilisierten Perchlorethylens wurden mit 0,1 Gew.-% Wasser versetzt und in Gegenwart von Stahlspänen (V2A-Stahl) 336 Stunden am Rückfluss gekocht. In dieser Anordnung war ferner ein Stahlblech so angeordnet, dass es zur Hälfte in die siedende Flüssigkeit eintauchte. Ein zweites V2A-Stahlblech war am Rückflusskühler so angebracht, dass das kondensierte Perchlorethylen über dieses Blech ablief.
Auch nach 336 Stunden waren die Bleche und Späne frei von Korrosionserscheinungen und Ablagerungen.

Vergleichsbeispiel 3
Es wurde die Arbeitsweise gemäss Beispiel 6 wiederholt, mit der Abänderung, dass ein Perchlorethylen verwendet wurde, das anstatt des Zusatzes von 100 Gew. ppm an erfindungsgemässen Epoxiden einen Zusatz von 200 Gew. ppm 2.3-Epoxipropanols enthielt.

Nach 336 Stunden war der in die Flüssigkeit eintauchende Stahlstreifen vollständig mit einem braunen Belag bedeckt.

## Patentanspruch

Verfahren zum Stabilisieren von Perchlorethylen, das als Stabilisierungsmittel Cyclohexenoxid enthält, dadurch gekennzeichnet, dass dem Perchlorethylen 4-Vinyl-cyclohexenmonoxid und/oder 7.8-Epoxiocten-1 und/oder 1.2-Epoxicycloocten-5 in Mengen von 0,5 bis 10 Gew.-%, bezogen auf die Menge anwesenden Cyclohexenoxids, zugesetzt werden.

## Revendication

Procédé de stabilisation de perchloréthylène contenant de l'oxyde de cyclohexène comme stabilisant, procédé caractérisé en ce que l'on ajoute au perchloréthylène du monoxyde de 4-vinyl-cyclohexène et/ou du 7,8-époxyoctène-1 et/ou du 1,2-époxycyclooctène-5 dans des proportions de 0,5 à 10% du poids de l'oxyde de cyclohexène présent.

## Claim

Process for stabilizing perchloroethylene which contains cyclohexene oxide as stabilizer, characterized in that 4-vinyl-cyclohexene monoxide and/or 7,8-epoxyoct-1-ene and/or 1,2-epoxyclooct-5-ene are added to the perchloroethylene in amounts of from 0.5 to 10% by weight, relative to the amount of cyclohexene oxide present.